# EUROPEAN PATENT SPECIFICATION

(11) **EP 0 631 500 B1**
(45) Date of publication and mention of the grant of the patent: **22.04.1998**
(21) Application number: 93905769.1
(22) Date of filing: 29.01.1993
(51) Int. Cl.: C07C 401/00, C07J 9/00, A61K 31/59

(54) **1 alpha-HYDROXY-24-(EPI)-VITAMIN D4**
1 alpha-HYDROXY-24-(EPI)-VITAMIN D4
1 alpha-HYDROXY-24-(EPI)-VITAMINE D4

(30) Priority: 29.01.1992 US 827173
(43) Date of publication of application: 04.01.1995
(73) Proprietor: BONE CARE INTERNATIONAL, INC., Madison, WI 53713 (US)
(72) Inventor: KNUTSON, Joyce, C., Madison, WI 53705 (US); MORIARTY, Robert, M., Oak Park, IL 60302 (US); PENMASTA, Raju, Elmhurst, IL 60126 (US); BISHOP, Charles, W., Verona, WI 53593 (US)
(74) Representative: Ford, Michael Frederick
(86) International application number: US9300796
(87) International publication number: WO9314763

(56) References cited:
- WO-A-92/05130
- US-A- 4 338 250
- US-A- 4 448 721
- US-A- 4 769 181
- US-A- 4 973 584
- BULLETIN DE LA SOCIETE CHIMIQUE DE FRANCE, no. 1, 1985 pages 98-114, R. PARDO AND M. SANTELLI 'Synthèse des métabolites de la vitamine D'
- SYNFORM, vol. 3, no. 2, May 1985 pages 75-76, P. J. KOCIENSKI ET AL 'Synthesis of 53 using two chiral building blocks, one obtained by partial synthesis and one prepared using a chirality inducing reaction.'
- CHEMICAL ABSTRACTS, vol. 111, no. 13, 25 September 1989 Columbus, Ohio, US; abstract no. 111630k, A. V. ANDREEV ET AL 'Products of the photoconversion of provitamin D4 obtained from mutants of yeast (Saccharomyces cerevisiae). I. Irradiation in ethanol.' page 333; column 1; & KHIM. PRIR. SOEDIN., no. 2, 1989 pages 247-254,
- Archives of Biochemistry and Biophysics, 1968, (DeLUCA et al.), "Synthesis, Biological Activity, and Metabolism of 22,23-H-Vitamin D4", pp. 122-128, esp. 122, 125-28.

## Description

This invention relates to biologically active vitamin D₄ compounds. More specifically, this invention relates to novel 1α-hydroxy-24-*epi*-vitamin D₄ and a method for preparing this compound as well as novel intermediates formed in the synthesis.

### BACKGROUND

The vitamins D are a group of compounds that are steroid derivatives and are known to be important in the regulation of calcium metabolism in animals and man. See, Harrison's Principles of Internal Medicine: Part Eleven, "Disorders of Bone and Mineral Metabolism, Chapter 335," E. Braunwald et al., (eds.), McGraw-Hill, New York, 1987, pp. 1860-1865.

The naturally occurring form of vitamin D in animals and man is vitamin D₃. Vitamin D₃ is synthesized endogenously in the skin of animals and man. In animals, including man, vitamin D₃ is activated by being hydroxylated in the C₂₅ position in the liver, followed by 1α-hydroxylation in the kidney to produce the hormone 1α,25-dihydroxy vitamin D₃. See, U.S. Patent No. 3,880,894.

1α,25-Dihydroxy vitamin D₃ is the hormonally active form of vitamin D₃. This hormone is taken up in the intestine by specific cytoplasmic receptor proteins to stimulate calcium and phosphate transport from the intestinal lumen to circulation. The vitamin D₃ hormone also is taken up by specific cytoplasmic receptors in the parathyroid glands, the kidney, the osteoblasts, and other target tissues, to elicit cellular responses which, synergistically, stabilize blood levels of calcium and phosphorus, control the formation and removal of bone, and regulate the further production of 1α,25-dihydroxy vitamin D₃ itself. It is now recognized that the 1α-hydroxy group is important in the binding of 1α,25-dihydroxy vitamin D₃ with its specific cytoplasmic receptors. It has also recently been reported that the vitamin D₃ hormones may play a role in cell proliferation and differentiation.

Vitamin D₂ is the major, naturally occurring form of vitamin D found in plants. Vitamin D₂ differs structurally from vitamin D₃ in that vitamin D₂ has a methyl group at C₂₄ and has a double bond between C₂₂ and C₂₃.

Considerable interest has focused on discovery and synthesis of various hydroxylated and dihydroxylated derivatives of vitamins D₃ and D₂. Examples of hydroxylated and dihydroxylated metabolites of vitamins D₃ and D₂ which have been found to occur naturally and/or have been synthesized include 25-hydroxy vitamin D₂, 24, 25-dihydroxy vitamin D₃, 25, 26-dihydroxy vitamin D₃, 1α-hydroxy vitamin D₂, 23, 25-dihydroxy vitamin D₃, all of which have been found to exhibit vitamin D-like biological activity in vivo.

Unfortunately, while many of these active vitamin D metabolites held great promise as therapeutic agents, this promise has never been fully realized because of the extreme toxicity of these agents. For example, toxicity limits the efficacy of vitamin D₃, its active forms and analogs, to prevent bone loss or restore lost bone. Many studies indicate that at dosages required for these agents to be effective in bone loss prevention or restoration, hypercalcemia and hypercalciuria are serious problems. It has been reported that 1α-hydroxy vitamin D₃ at a daily dose of 2 µg/day (which has been shown in some studies to be effective in preventing loss of bone) causes toxicity in approximately 67 percent of patients.

Vitamin D₄ is a little known form of vitamin D. Vitamin D₄ was first described in 1936. See, Grab, W., Z. Physiol. Chem., 243:63 (1936); McDonald, F. G., J. Biol. Chem., 114:IVX (1936). See also, Windaus, A. and Trautmann, G., Z. Physiol. Chem., 247:185-188 (1937). Vitamin D₄, also known as irradiated 22,23-dihydro-ergosterol or 22,23-dihydro vitamin D₂ or 22,23-dihydroergocalciferol, differs from vitamin D₃ in that it contains a C₂₄ methyl group. The above cited references disagree as to the level of biological activity of this D vitamin, suggesting that in the rat, vitamin D₄ is one-third or three-fourths as active as vitamin D₃, and in the chick, either one-tenth or one-fifth as active as vitamin D₃.

In 1968, DeLuca et al. (Arch. Biochem. Biophys., 124:122-128 (1968)) confirmed that vitamin D₄ was less active than vitamin D₃. DeLuca et al. reported that vitamin D₄ is two-thirds as active as vitamin D₃ or vitamin D₂ in the rat, and one-fifth as active as vitamin D₃ in the chick.

DeLuca et al. make reference to the fact that "[t]he synthesis of vitamin D₄ has apparently been little used since it was first described by Windaus and Trautmann," and comment, "[t]his is perhaps due to the fact that vitamin D₄ is only of academic interest."

To applicants' knowledge, vitamin D₄ has remained "only of academic interest" as applicants are unaware of any further study of vitamin D₄ since that reported by DeLuca et. al. In fact, The Merck Index states with respect to vitamin D₄, "[i]ts biological activity seems doubtful." Merck Index, S. Budavari (ed.), 11th ed., Merck & Co., Rahway, N.J., (1989) pp. 1579, #9930.

There has been even less interest in vitamin D₄ analogues. Recently, however, a vitamin D₄ analogue, 1α-hydroxy vitamin D₄, has been synthesized and shown to possess unexpectedly high biopotency and low toxicity (co-pending U.S. Patent Application Serial No. 07/586,854, filed September 21, 1990). It was surprising to applicants in that application that this vitamin D₄ analogue had activity commensurate with the vitamin D₃ and D₂ hormones. Applicants, in this invention, have synthesized a related isomer of la-hydroxy vitamin D₄ with equally surprising biological activity.

### SUMMARY OF THE INVENTION

The present invention provides a stereoisomer of vitamin D₄, 1α-hydroxy-24-*epi*-vitamin D₄, tosylated and cyclic derivatives of this compound, and a method of preparing these compounds.

In one aspect, the invention provides the compounds of formula (I) as defined hereinbelow. 1α-Hydroxy-24-*epi*-vitamin D₄, the compound of formula (I) wherein R₁ and R₂ are each hydroxy groups, has been found to be bioactive. Other compounds encompassed by formula (I) have been found to be novel intermediates in the synthesis of 1α-hydroxy-24-*epi*-vitamin D₄.

In another aspect, the invention provides the compounds of formula (II) which have also been found to be novel intermediates in the synthesis of 1α-hydroxy-24-*epi*-vitamin D₄.

In further aspect, the invention provides a synthetic route for making the 1α-hydroxy-24-*epi*-vitamin D₄. The method includes campesterol as a starting material which is converted to 24-*epi*-vitamin D₄ which is in turn hydroxylated to 1α-hydroxy-24-*epi*-vitamin D₄ via tosylated and cyclic derivatives of 24-epi-vitamin D₄. A novel intermediate which is a derivative of campesterol has also been found.

Other advantages and a fuller appreciation of the specific adaptations, compositional variations, and physical and chemical attributes of the present invention will be gained upon an examination of the following detailed description of the invention, taken in conjunction with the accompanying drawings.

### BRIEF DESCRIPTION OF THE DRAWINGS

The present invention will hereinafter be described and exemplified in conjunction with the appended drawings, wherein like designations refer to like elements throughout and in which:
Figure 1 illustrates preparative steps for the synthesis of 24-*epi*-vitamin D₄ starting with campesterol; and
Figure 2 illustrates preparative steps for the synthesis of 1α-hydroxy-24-*epi*-vitamin D₄ starting with 24-*epi*-vitamin D₄.

### DETAILED DESCRIPTION

The present invention provides synthetic 1α-hydroxy-24-*epi*-vitamin D₄ (1α-OH-24-*epi*-D₄) as well as tosylated and cyclic derivatives of this compound.

As used herein, the terms "biological activity" or "biologically active" are meant to refer to biochemical properties of compounds such as affecting metabolism, e.g., affecting serum calcium concentration, or binding to an appropriate receptor protein, e.g., binding to vitamin D receptor protein. The term *"epi"* as used herein and as used generally in the art is meant to designate a different absolute configuration about a carbon atom, in the present invention, about the C₂₄ carbon, than in the parent vitamin D₄ structure.

In one of its aspects, the invention encompasses the compounds of the general formula (I): 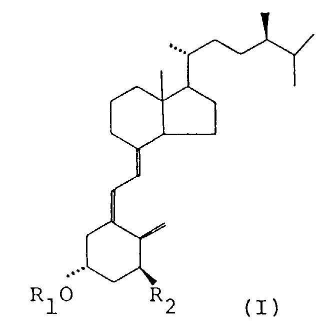 wherein R₁ is hydrogen or tosyl and R₂ is hydrogen or hydroxy, and salts, hydrates and solvates thereof. Preferred among those compounds of formula (I) is that in which R₁ is hydrogen and R₂ is OH, i.e., 1α-hydroxy-24-*epi*-vitamin D₄, which has been found to increase serum calcium.

In another aspect, the invention provides compounds of formula (II): 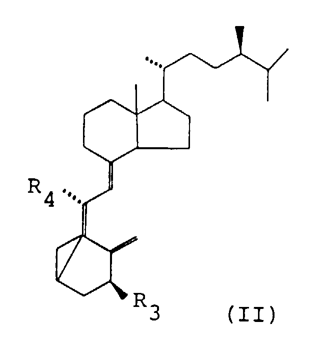 wherein R₃ is either hydrogen or hydroxy, and R₄ is methoxy, and salts, hydrates and solvates thereof. These compounds have been found to be useful and novel intermediates to form 1α-hydroxy-24-*epi*-vitamin D₄.

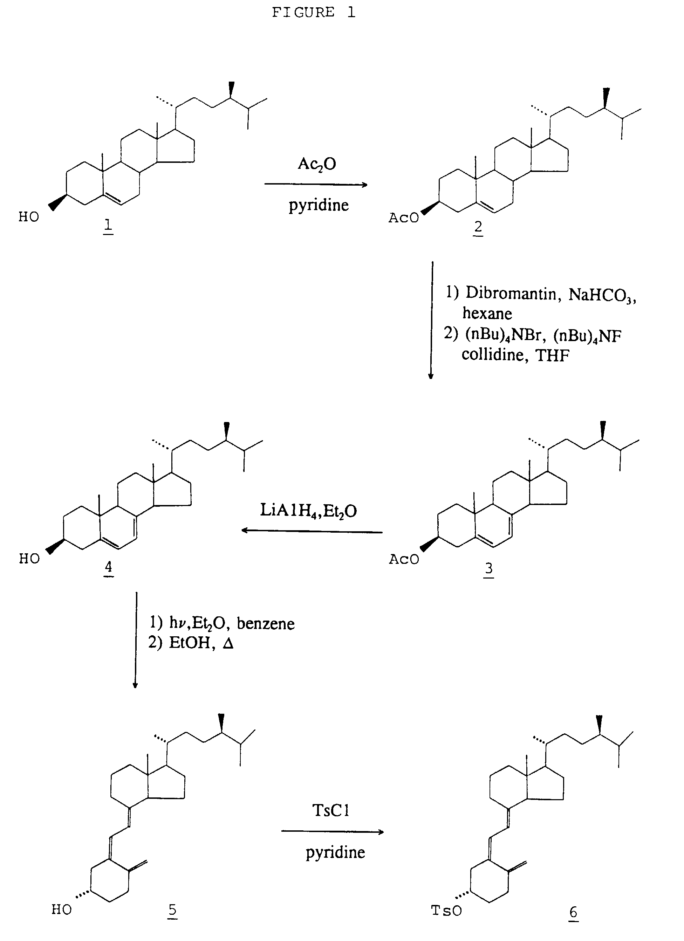

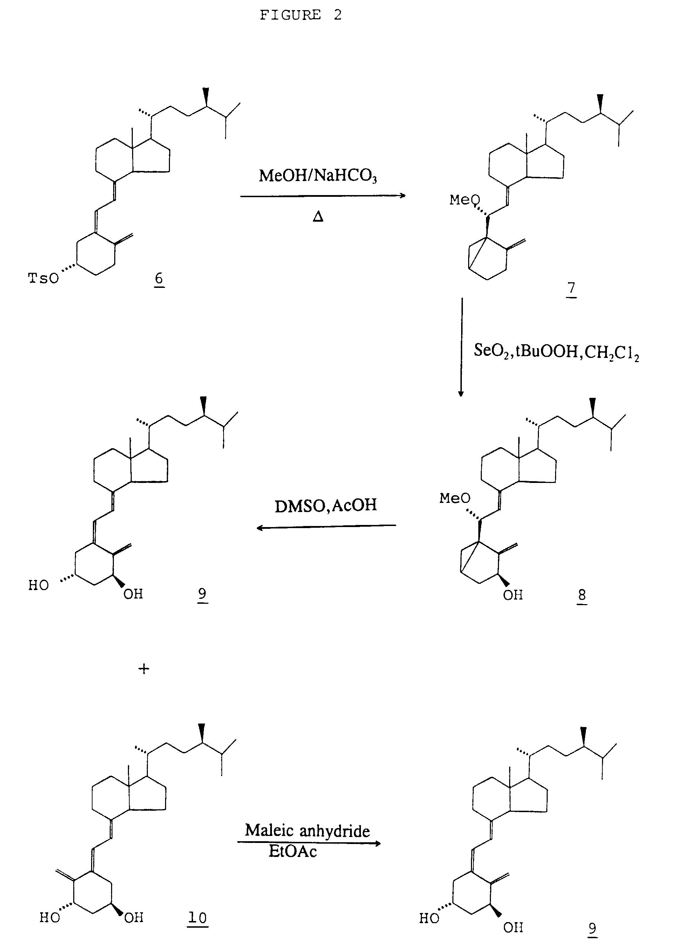

In still another aspect, the invention involves the preparation of compounds of formulas (I) and (II) as well as another novel intermediate. Specifically, the synthesis of 1α-hydroxy-24-epi-vitamin D₄, i.e., the compound of formula (I) wherein R₁ is hydrogen and R₂ is OH, is accomplished according to the schema presented in Figures 1 and 2. As seen in Figure 1, the synthesis uses the steroid campesterol as the starting material. Campesterol is available according to the procedure of Tarzia et al., Gazz. Chem. Ital., vol. 97, pp. 102-106 (1967). Campesterol undergoes C₇ bromination, C₇-C₈ dehydrobromination in a four-step process to yield 7-dehydrocampersterol. The 7-dehydrocampesterol is then irradiated and thermally converted by methods well known in the art to yield 24-*epi*-vitamin D₄ [also known as 22,23-dihydro-24-*epi*-ergocalciferol]. As seen in Figure 2, 24-*epi*-vitamin D₄ is then hydroxylated in a five-step process to yield 1α-hydroxy-24-*epi*-vitamin D₄.

Specifically, campesterol is acetylated to form the 3β-acetate. This campesterol acetate is subjected to C₇ bromination, C₇-C₈ dehydrobromination to form a double bond at C₇-C₈. The resulting 7-dehydrocampesterol acetate is then reduced to the novel 7-dehydrocampesterol. The 7-dehydrocampesterol is then irradiated and thermally converted to yield 24-*epi*-vitamin D₄. The 24-epi-vitamin D₄ is then tosylated to yield the 3β-tosylate of 24-*epi*-vitamin D₄. The tosylate is displaced by solvolysis to yield the 6-methoxylate of 24-*epi*-3,5-cyclovitamin D₄. This 24-*epi*-cyclovitamin D₄ is subjected to allylic oxidation to form the la-hydroxy 24-*epi*-cyclovitamin derivative. The 1α-hydroxy 24-*epi*-cyclovitamin derivative is sequentially hydrolyzed and subjected to a Diels-Alder type reaction which removes the 6-methoxy group and separates the 1α-hydroxy 24-*epi*-vitamin D₄ (5,6 cis) from the 5,6 trans 1α-hydroxy 24-epi-vitamin D₄. It is noted that the trans isomer, if desired, may be separated from the cis isomer via high pressure liquid chromatography according to the procedure disclosed, for example, in U.S. Patent 4,719,204 issued to DeLuca et al.

1α-Hydroxy-24-*epi*-vitamin D₄ has been found to possess physiological activity, namely, as an agent for increasing serum calcium concentrations. Specifically, this compound increases serum calcium concentrations in rats with vitamin D deficiency. Thus, the compounds of the invention are potentially applicable to various clinical and veterinary fields, and are particularly useful for the treatment of abnormal metabolism of calcium and phosphorus.

The following examples are to be construed as merely illustrative, and not limitative of the remainder of the disclosure in any way whatsoever. In the following examples, all temperatures are set forth in degrees Celsius; unless otherwise indicated, all product yields are reported as percentages by weight. Proton nuclear magnetic resonance (¹H NMR) spectra were recorded with a Bruker AM--400(400 MHz) with aspect 3000 Computer in CDCl₃ solutions with CHCl₃ as an internal standard. Chemical shifts are reported in ppm. Ultraviolet spectra were recorded with a Hitachi U-2000 Spectrophotometer and are reported for ethanol solutions.

### Example 1: Synthesis of 1α-hydroxy-24-epi-vitamin D₄

### Campesterol Acetate (2):

To a solution of 24.0 g (0.06 mol) of campesterol (1) in 180 ml of anhydrous pyridine was added 18.5 ml (0.196 mol) of acetic anhydride. The mixture was stirred at room temperature overnight and then 600 ml of water was added. The precipitate was filtered and washed three times with 200 ml portions of acetonitrile, and then air dried to yield 20.0 g (75%) of (2).

¹H NMR: (400 MHz, CDCl₃); δppm 0.7 (3H, s, 18-CH₃), 0.8 (6H, dd, 26 and 27-CH₃), 0.86 (3H, d, 21-CH₃), 0.92 (3H, d, 28-CH₃), 1.02 (3H, s, 19-CH₃), 2.04 (3H, s, OCOCH₃), 4.6 (1H, m, 3-H), 5.38 (1H, m, 6-H).

### 7-Dehydrocampesterol acetate (3)

A mixture of 10 g (0.023 mol) of (2), 4.56 g (0.016 mol) of dibromantin and 10.2 g (0.121 mol) of anhydrous sodium bicarbonate in 250 ml of dry hexane was heated under reflux in a nitrogen atmosphere for 2 hrs. The precipitate was filtered off and the solution was concentrated to dryness under reduced pressure. To the solution of the residue in 50 ml of anhydrous tetrahydrofuran was added 0.65 g (2.02 mmol) of tetrabutylammonium bromide, and the mixture was stirred at room temperature for 30 min under nitrogen. A solution of tetrabutylammonium fluoride (112 ml, 1M in THF) was then added followed by 5.0 ml of s-collidine, and the mixture was stirred under nitrogen at room temperature overnight. To this reaction mixture was added ether (700 ml), and the organic phase was washed with water (2x200 ml), cold 1M HCl (2x200 ml) and 10% sodium bicarbonate (2x200 ml), and dried over anhydrous MgSO₄. Chromatography on silica gel with 10% ethyl acetate in hexane gave 5.5 g (55%) of (3).

¹H NMR: (400 MHz, CDCl₃); δppm 0.62 (3H, s, 18-CH₃), 0.80 (6H, dd, 26 and 27-CH₃), 0.86 (3H, d, 21-CH₃), 0.94 (3H, d, 28-CH₃), 0.96 (3H, s, 19-CH₃), 2.05 (3H, s, OCOCH₃), 4.7 (1H, m 3-H), 5.4 (1H, m, 7-H), 5.58 (1H, m, 6-H).

### 7-Dehydrocampesterol (4)

To a solution of 5.5 g (0.012 mol) of (3) in dry ether (500 ml) was added 3.38 g (0.089 mol) of lithium aluminum hydride. The mixture was stirred at room temperature for 2 hours, cooled with an ice water bath, and the reaction mixture decomposed by the cautious dropwise addition of ice water (5 ml). The mixture was filtered and the filtrate was concentrated *in vacuo* to remove most of the tetrahydrofuran. The residue was dissolved in 1000 ml of ether and washed with saturated NaCl solution (2x500 ml), dried over anhydrous MgSO₄ and concentrated *in vacuo.* The residue was purified on a silica gel column using 20% ethyl acetate in hexane to yield 4.0 g (80%) of (4).

¹H NMR: (400 MHz, CDCl₃); δppm 0.62 (3H, s, 18-CH₃), 0.8 (6H, dd, 26 and 27-CH₃), 0.86 (3H, d, 21-CH₃), 0.94 (3H, d, 28-CH₃), 0.96 (3H, s, 19-CH₃), 3.62 (1H, m, 3-H), 5.39 (1H, m, 7-H), 5.58 (1H, m, 6-H).

### 24-epi-Vitamin D₄ (5)

7-Dehydrocampesterol (4) (3.0 g, 7.5 mmol) was dissolved in 500 ml of ether and benzene (4:1) and irradiated with stirring under nitrogen in a water-cooled quartz immersion well using a Hanovia medium-pressure UV lamp for 1.5 hrs. The solution was concentrated *in vacuo,* redissolved in 200 ml of ethanol and heated under reflux overnight. The solution was concentrated to dryness *in vacuo* and the residue was purified on a silica gel column using 20% ethyl acetate in hexane to yield 0.9 g (30%) of (5).

¹H NMR: (400 MHz, CDCl₃); δppm 0.54 (3H, s, 18-CH₃), 0.76 (6H, dd, 26 and 27-CH₃), 0.82 (3H, d, 21-CH₃), 0.9 (3H, d, 28-CH₃), 3.91 (1H, m, 3-H), 4.7 (1H, m, 19-H), 5.03 (1H, m, 19-H), 6.02 (1H, d, 7-H), 6.21 (1H, d, 6-H). UV (ethanol) λₘₐₓ: 265 nm.

### 24-epi Vitamin-D₄ tosylate (6)

To a solution of 0.9 g (2.26 mmol) of (5) dissolved in 10 ml of anhydrous pyridine was added 1.2 g (6.30 mmol) of tosyl chloride. The mixture was stirred under nitrogen at 5°C for 24 hrs. The reaction mixture was poured into 100 ml of cold saturated NaHCO₃ solution and extracted with ether (3x200 ml). The combined ether extracts were washed with 5% HCl solution (3x300 ml), saturated sodium bicarbonate solution (3x300 ml) and saturated NaCl solution (2x300 ml), dried over anhydrous MgSO₄ and concentrated *in vacuo* to yield 1.1 g (88%) of (6).

### 24-epi-3,5-Cyclovitamin D₄ (7)

To a solution of 1.0 g (1.81 mmol) of (6) dissolved in 100 ml of anhydrous methanol was added sodium bicarbonate 10.0 g (0.12 mol). The mixture was heated under reflux for 8 hrs. The reaction mixture was concentrated *in vacuo.* Water (200 ml) was added followed by extraction with ether (2x300 ml). The combined ether extracts were dried over anhydrous MgSO₄ and concentrated to dryness *in vacuo* to yield 600 mg (80%) of (7) as an oil.

¹H NMR: (400 MHz, CDCl₃); δppm 0.54 (3H, s, 18-CH₃), 0.78 (6H, dd, 26 and 27-CH₃), 0.86 (3H, d, 21-CH₃), 0.92 (3H, d, 28-CH₃), 3.25 (3H, s, -OCH₃), 4.16 (1H, d, 6-H), 4.86 (1H, m, 19-H), 4.98 (1H, d, 7-H), 5.02 (1H, m, 19-H).

### 1α-Hydroxy-24-epi-3,5-cyclovitamin D₄ (8)

*tert*-Butyl hydroperoxide (1.13 ml, 3.39 mmol; 3M in toluene) was added to a suspension of 95 mg (0.86 mmol) of selenium dioxide in 65 ml of anhydrous dichloromethane under nitrogen. The mixture was stirred at room temperature under nitrogen for 3 hours. Then 0.13 ml of anhydrous pyridine was added followed by a solution of 600 mg (1.45 mmol) of (7) dissolved in 20 ml of anhydrous dichloromethane. The mixture was stirred under nitrogen at room temperature for 15 min, then 25 ml of 10% NaOH solution was added and the mixture was extracted with ether (3x100 ml). The combined ether extracts were washed with 10% NaOH solution (3x100 ml), water (3x100 ml), saturated sodium chloride solution (2x100 ml), dried over anhydrous MgSO₄ and concentrated to dryness *in vacuo.* The residue was purified on a silica gel column using a mixture of 20% ethyl acetate in hexane to yield 140 mg (23%) of (8) as an oil.

¹H NMR: (400 MHz, CDCl₃); δppm, 0.54 (3H, s, 18-CH₃), 0.79 (6H, dd, 26 and 27-CH₃), 0.88 (3H, d, 21-CH₃), 0.92 (3H, d, 28-CH₃), 3.24 (3H, s, -OCH₃) 4.2 (1H, m, 3-H), 4.21 (1H, d, 6-H), 4.94 (1H, d, 7-H), 5.15 (1H, m, 19-H), 5.21 (1H, m, 19-H).

### 5.6-cis and 5.6-trans-1α-hydroxy-24-epi-vitamin D₄ (9, 10)

1α-Hydroxy-24-*epi*-3,5 cyclovitamin D₄ (8) (110 mg, 0.26 mmol) was dissolved in 1.1 ml of dimethylsulfoxide and 0.9 ml of acetic acid and heated at 50°C under nitrogen for 1 hour. The solution was poured over ice and 50 ml of saturated NaHCO₃ solution. The mixture was extracted with ether (3x100 ml). The combined ether extracts were washed with saturated NaHCO₃ solution (3x100 ml), water (2x100 ml), and saturated NaC1 solution (2x200 ml), dried over anhydrous MgSO₄ and concentrated *in vacuo* to yield the crude product 105 mg (95%) of (9) and (10).

### 5.6-cis-1α-hydroxy-24-epi-vitamin D₄ (9)

To a solution of (9) and (10), 105 mg (0.25 mmol) in 5 ml of ethyl acetate, was added 20 mg (0.2 mmol) of maleic anhydride, and the mixture was stirred at 35°C for 24 hours under nitrogen. The solution was concentrated to dryness *in vacuo.* The residue was purified on a silica gel column using 40% ethyl acetate in hexane to yield 30 mg (28%) of (9).

¹H NMR: (400 MHz, CDCl₃); δppm 0.54 (3H, 1 s, 18-CH₃), 0.78 (6H, dd, 26 and 27-CH₃), 0.86 (3H, d, 21-CH₃), 0.92 (3H, d, 28-CH₃), 4.2 (1H, m, 3-H), 4.41 (1H, m, 1-H), 5.0 (1H, m, 19-H), 5.32 (1H, m, 19-H), 6.0 (1H, m, 7-H), 6.38 (1H, m, 6-H); UV (ethanol) λₘₐₓ: 265 nm.

### Example 2: Biological testing of 1α-hydroxy-24-epi-vitamin D₄

Male weanling rats (Holtzman strain, Holtzman Company, Madison, Wisconsin) were fed a vitamin D deficient diet containing adequate calcium (0.47%) and phosphorus (0.3%). Within three to four weeks, this diet induces an extreme vitamin D deficiency characterized by low serum calcium and poor growth. After four weeks on this diet, the rats had serum calcium values less than 6 mg/dl. The rats were then separated into four groups and orally administered either 1α-hydroxy-24-*epi*-vitamin D₄ in a vehicle such as coconut oil or the vehicle (control) for each of 14 days. Twenty-four hours after the last dose, the rats were killed, and the blood calcium measured by a standard laboratory technique. The results of these determinations are shown in Table 1.

**Table 1**

| Increase in serum calcium concentration | | | |
|---|---|---|---|
| Compound | Dose (mcg/kg/day) | Number of Rats | Serum Calcium Concentration (mg/100 ml) ± Standard Deviation |
| Vehicle | - | 10 | 5.1 ± 0.42 |
| 24-*epi*-1α-OH-D₄ | 0.042 | 11 | 5.8 ± 0.40 |
| 24-*epi*-1α-OH-D₄ | 0.250 | 12 | 8.1 ± 1.25 |
| 24-*epi*-1α-OH-D₄ | 1.500 | 12 | 10.5 ± 0.71 |

The data of Table 1 indicate that 1α-hydroxy-24-*epi*-vitamin D₄ is effective at increasing serum calcium in the vitamin D deficient rat and that the response appears to be dose dependent.

## Claims

1. The compound of the formula (I) : 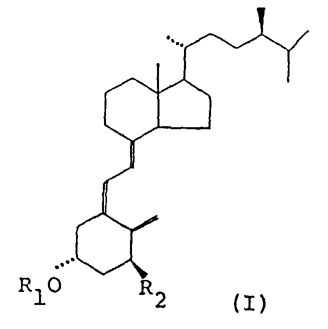 wherein R₁ is either hydrogen or tosyl and R₂ is either hydrogen or hydroxy, and salts, hydrates and solvates thereof.

2. The compound of claim 1, wherein the compound is 1α-hydroxy-24-*epi*-vitamin D₄.

3. The compound of claim 1, wherein the compound is 24-*epi*-vitamin D₄ tosylate.

4. The compound of the formula (II): 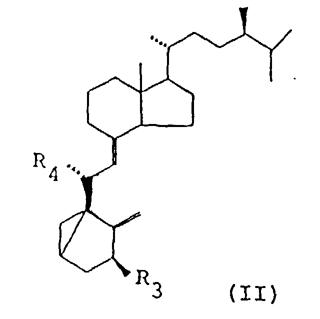 wherein R₃ is hydrogen or hydroxy and R₄ is methoxy.

5. The compound of claim 4, wherein the compound is 24-*epi*-3,5-cyclovitamin D₄.

6. The compound of claim 4, wherein the compound is 1α-hydroxy-24-*epi*-3,5-cyclovitamin D₄.

7. 7-Dehydrocampesterol.

8. 5,6-trans-1α-hydroxy-24-*epi*-vitamin D₄.

9. A method of producing 24-*epi*-vitamin D₄ tosylate, comprising reacting 24-*epi*-vitamin D₄ with toluenesulfonyl chloride in the presence of dry pyridine.

10. A method of producing 24-*epi*-3,5-cyclovitamin D₄, comprising subjecting 24-*epi*-vitamin D₄ tosylate to buffered solvolysis.

11. A method of producing 1α-hydroxy-24-*epi*-3,5-cyclovitamin D₄, comprising allylically oxidizing the 24-*epi*-3,5-cyclovitamin D₄ with selenium dioxide.

12. A method of producing 1α-hydroxy-24-*epi*-vitamin D₄, comprising hydrolyzing the 1α-hydroxy-24-*epi*-3,5 cyclovitamin D₄ with a mixture of dimethylsulfoxide and an organic acid to form an admixture of the 5,6 cis 1α-hydroxy-24-epi-vitamin D₄ and 5,6 trans 1α-hydroxy-24-*epi*-vitamin D₄ and subjecting the admixture to a Diels-Alder reaction forming an adduct of the 5,6 trans 1α-hydroxy-24-*epi*-vitamin D₄ to allow purification to yield the 1α-hydroxy-24-*epi*-vitamin D₄.

13. The method of claim 12 preceded by:
(a) tosylating 24-*epi*-vitamin D₄ in the presence of dry pyridine to form 24-*epi*-vitamin D₄ tosylate;
(b) solvolyzing 24-*epi*-vitamin D₄ tosylate to form 24-*epi*-3,5 cyclovitamin D₄; and
(c) allylically oxidizing the 24-*epi*-3,5 cyclovitamin D₄ with selenium dioxide to form said 1α-hydroxy-24-*epi*-3,5-cyclovitamin D₄.

14. A method of producing 1α-hydroxy-24-*epi*-vitamin D₄, comprising: oxidizing campesterol to form 7-dehydrocampesterol; irradiating the 7-dehydrocampesterol to form 24-*epi*-vitamin D₄; and hydroxylating 24-*epi*-vitamin D₄ to form 1α-hydroxy-24-*epi*-vitamin D₄.

15. The method of claim 14 wherein oxidation of campesterol is carried out by:
acetylating campesterol to form campesterol acetate;
oxidizing the campesterol acetate to form 7-dehydrocampesterol acetate; and
reducing the 7-dehydrocampesterol acetate to 7-dehydrocampesterol;
and hydroxylation of 24-*epi*-vitamin D₄ is carried out by:
tosylating 24-*epi*-vitamin D₄ in the presence of dry pyridine to form 24-*epi*-vitamin D₄ tosylate;
solvolyzing 24-*epi*-vitamin D₄ tosylate to form 24-*epi*-3,5-cyclovitamin D₄;
allylically oxidizing the 24-*epi*-3,5-cyclovitamin D₄ with selenium dioxide to form 1α-hydroxy-24-*epi*-3,5-cyclovitamin D₄; and
hydrolyzing the 1α-hydroxy-24-*epi*-3,5-cyclovitamin D₄ with a mixture of dimethylsulfoxide and an organic acid to form an admixture of the 5,6 cis 1α-hydroxy-24-*epi*-vitamin D₄ and 5,6 trans 1α-hydroxy-24-*epi*-vitamin D₄ and forming a Diels-Alder adduct of the 5,6 trans 1α-hydroxy-24-*epi*-vitamin D₄ to allow purification to yield 1α-hydroxy-24-*epi*-vitamin D₄.

## Patentansprüche

1. Verbindung der Formel (1): 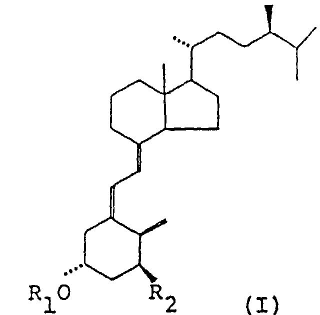 worin R₁ entweder Wasserstoff oder Tosyl ist und R₂ entweder Wasserstoff oder Hydroxy ist, sowie Salze, Hydrate und Solvate davon.

2. Verbindung nach Anspruch 1, worin die Verbindung 1α-Hydroxy-24-epi-vitamin D₄ ist.

3. Verbindung nach Anspruch 1, worin die Verbindung 24-Epi-vitamin D₄-tosylat ist.

4. Verbindung der Formel (II): 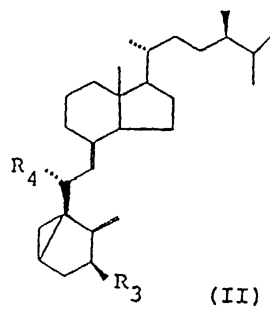 worin R₃ Wasserstoff oder Hydroxy ist und R₄ Methoxy ist.

5. Verbindung nach Anspruch 4, worin die Verbindung 24-Epi-3,5-cyclovitamin D₄ ist.

6. Verbindung nach Anspruch 4, worin die Verbindung 1α-Hydroxy-24-epi-3,5-cyclovitamin D₄ ist.

7. 7-Dehydrocampesterol.

8. 5,6-trans-1α-Hydroxy-24-epi-vitamin D₄.

9. Verfahren zur Herstellung von 24-Epi-vitamin D₄-tosylat, umfassend das Umsetzen von 24-Epi-vitamin D₄ mit Toluolsulfonylchlorid in Gegenwart von trockenem Pyridin.

10. Verfahren zur Herstellung von 24-Epi-3,5-cyclovitamin D₄, umfassend die Anwendung von gepufferter Solvolyse auf 24-Epi-vitamin D₄.

11. Verfahren zur Herstellung von 1α-Hydroxy-24-epi-3,5-cyclovitamin D₄, umfassend die Allyloxidation von 24-Epi-3,5-cyclovitamin D₄ mit Selendioxid.

12. Verfahren zur Herstellung von 1α-Hydroxy-24-epi-vitamin D₄, umfassend die Hydrolyse von 1α-Hydroxy-24-epi-3,5-cyclovitamin D₄ mit einem Gemisch aus Dimethylsulfoxid und einer organischen Säure, um ein Gemisch aus 5,6-cis-1α-Hydroxy-24-epi-vitamin D₄ und 5,6-trans-1α-Hydroxy-24-epi-vitamin D₄ zu bilden, und die Durchführung einer Diels-Alder-Reaktion mit dem Gemisch, wodurch ein Addukt des 5,6-trans-1α-Hydroxy-24-epi-vitamins D4 gebildet wird, um eine Reinigung zu ermöglichen, um das 1α-Hydroxy-24-epi-vitamin D₄ zu erhalten.

13. Verfahren nach Anspruch 12, dem vorangeht:
(a) die Tosylierung von 24-Epi-vitamin D₄ in Gegenwart von trockenem Pyridin, um 24-Epi-vitamin D₄-tosylat zu bilden;
(b) die Solvolyse von 24-Epi-vitamin D₄-tosylat, um 24-Epi-3,5-cyclovitamin D₄ zu bilden; und
(c) die Allyloxidation des 24-Epi-3,5-cyclovitamins D₄ mit Selendioxid, um das 1α-Hydroxy-24-epi-3,5-cyclovitamin D₄ zu bilden.

14. Verfahren zur Herstellung von 1α-Hydroxy-24-epi-vitamin D₄, umfassend: die Oxidation von Campesterol, um 7-Dehydrocampesterol zu bilden; Bestrahlung des 7-Dehydrocampesterols, um 24-Epi-vitamin D₄ zu bilden; und Hydroxylierung von 24-Epi-vitamin D₄, um 1α-Hydroxy-24-epi-vitamin D₄ zu bilden.

15. Verfahren nach Anspruch 14, worin die Oxidation von Campesterol folgendermaßen erfolgt:
Acetylierung von Campesterol, um Campesterolacetat zu bilden;
Oxidation des Campesterolacetats, um 7-Dehydrocampesterolacetat zu bilden; und
Reduktion des 7-Dehydrocampesterolacetats zu 7-Dehydrocampesterol; und die Hydroxylierung von 24-Epi-vitamin D₄ folgendermaßen erfolgt:
Tosylierung von 24-Epi-vitamin D₄ in Gegenwart von trockenem Pyridin, um 24-Epi-vitamin D₄-tosylat zu bilden;
Solvolyse von 24-Epi-vitamin D₄-tosylat, um 24-Epi-3,5-cyclovitamin D₄ zu bilden;
Allyloxidation des 24-Epi-3,5-cyclovitamins D₄ mit Selendioxid, um 1α-Hydroxy-24-epi-3,5-cyclovitamin D₄ zu bilden; und
Hydrolyse des 1α-Hydroxy-24-epi-3,5-cyclovitamins D₄ mit einem Gemisch aus Dimethylsulfoxid und einer organischen Säure, um ein Gemisch aus 5,6-cis-1α-Hydroxy-24-epi-vitamin D₄ und 5,6-trans-1α-Hydroxy-24-epi-vitamin D₄ zu bilden, und Bildung eines Diels-Alder-Adduktes des 5,6-trans-1α-Hydroxy-24-epi-vitamins D₄, um eine Reinigung zu ermöglichen, um 1α-Hydroxy-24-epi-vitamin D₄ zu erhalten.

## Revendications

1. Composé de la formule (I) : 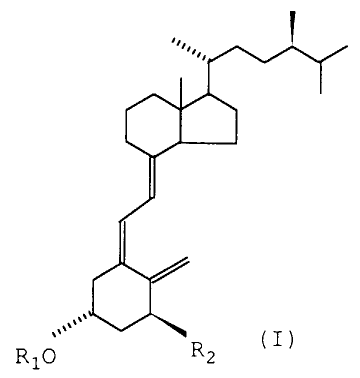 où R₁ est soit de l'hydrogène ou du tosyle et R₂ est soit de l'hydrogène ou un hydroxy, et sels, hydrates et solvates.

2. Composé de la revendication 1, où le composé est 1α-hydroxy-24-*épi*-vitamine D₄.

3. Composé de la revendication 1, où le composé est le tosylate de 24-*épi*-vitamine D₄.

4. Composé de la formule (II) : 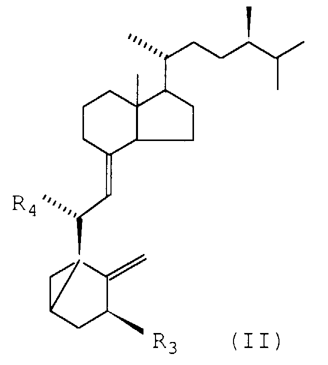 où R₃ est hydrogène ou hydroxy et R₄ est méthoxy.

5. Composé de la revendication 4, où le composé est 24-épi-3,5-cyclovitamine D₄.

6. Composé de la revendication 4, où le composé est 1α-hydroxy-24-*épi*-3,5-cyclovitamine D₄.

7. 7-Déshydrocampestérol.

8. 5,6-trans-1α-hydroxy-24-*épi*-vitamine D₄.

9. Méthode de production du tosylate de 24-*épi*-vitamine D₄, comprenant la réaction de 24-*épi*-vitamine D₄ avec du chlorure de toluènesulfonyle en présence de pyridine sèche.

10. Méthode de production de 24-*épi*-3,5-cyclovitamine D₄, consistant à soumettre le tosylate de 24-*épi*-vitamine D₄ à une sovolyse tamponnée.

11. Méthode de production de 1α-hydroxy-24-*épi*-3,5-cyclovitamine D₄, comprenant l'oxydation allylique de la 24-*épi*-3,5-clyclovitamine D₄ avec du dioxyde de sélénium.

12. Méthode de production de 1α-hydroxy-24-*épi*-vitamine D₄, comprenant l'hydrolyse de la 1α-hydroxy-24-*épi*-3,5 cyclovitamine D₄ avec un mélange de diméthylsulfoxyde et d'un acide organique pour former un mélange de 5,6 cis 1α-hydroxy-24-*épi*-vitamine D₄ et de 5,6 trans 1α-hydroxy-24-*épi*-vitamine D₄ et la soumission du mélange à une réaction de Diels-Alder pour former un produit d'addition de 5,6 trans 1α-hydroxy-24-*épi*-vitamine D₄ pour permettre à la purification de donner la 1α-hydroxy-24-*épi*-vitamine D₄.

13. Méthode de la revendication 12 précédée de :
(a) la tosylation de 24-*épi*-vitamine D₄ en présence de pyridine sèche pour former le tosylate de 24-*épi*-vitamine D₄;
(b) la solvolyse du tosylate de 24-épi-vitamine D₄ pour former la 24-*épi*-3,5 cyclovitamine D₄; et
(c) l'oxydation allylique de 24-*épi*-3,5-cyclcvitamine D₄ avec du dioxyde de sélénium pour former ladite 1α-hydroxy-24-*épi*-3,5-cyclovitamine D₄.

14. Méthode de production de 1α-hydroxy-24-*épi*-vitamine D4 comprenant : l'oxydation du campestérol pour former le 7-déshydrocampestérol; l'irradiation du 7-déshydrocampestérol pour former la 24-*épi*-vitamine D₄; et l'hydroxylation de la 24-*épi*-vitamine D₄ pour former la 1α-hydroxy-24-*épi*-vitamine D₄.

15. Méthode de la revendication 14 où l'oxydation du campestérol est effectuée par :
l'acétylation du campestérol pour former l'acétate de campestérol;
l'oxydation de l'acétate de campestérol pour former l'acétate de 7-déshydroxycampestérol; et
la réduction de l'acétate de 7-déshydroxycampestérol en 7-déshydrocampestérol;
et l'hydroxylation de la 24-*épi*-vitamine D₄ est effectuée par :
la tosylation de la 24-*épi*-vitamine D₄ en présence de pyridine sèche pour former le tosylate de 24-*épi*-vitamine D₄;
la solvolyse de tosylate de 24-*épi*-vitamine D₄ pour former la 24-*épi*-3,5-cyclovitamine D₄; et
l'oxydation allylique de la 24-*épi*-3,5-cyclovitamine D₄ avec du dioxyde de sélénium pour former la 1α-hydroxy-24-*épi*-3,5-cyclovitamine D₄; et
l'hydrolyse de la 1α-hydroxy-24-*épi*-3,5-cyclovitamine D₄ avec un mélange de diméthylsulfoxyde et d'un acide organique pour former un mélange de la 5,6 cis 1α-hydroxy-24-*épi*-vitamine D₄ et de la 5,6 trans 1α-hydroxy-24-*épi*-vitamine D₄ et la formation un produit d'addition de Diels-Alder de la 5,6 trans 1α-hydroxy-24-*épi*-vitamine D₄ pour permettre à la purification de donner la 1α-hydroxy-24-*épi*-vitamine D₄.
